# EUROPEAN PATENT APPLICATION

(11) **EP 1 774 980 A1**
(43) Date of publication of application: **18.04.2007**
(21) Application number: 06255187.4
(22) Date of filing: 09.10.2006
(51) Int. Cl.: A61L 2/18, A61L 2/232, A61L 2/26, B32B 7/00, A01N 25/34, C11D 17/04, A61L 2/28, A61L 101/32

(54) **Dual-sided cleansing wipes for medical equipment**

(30) Priority: 11.10.2005 US 247615
(71) Applicant: General Electric Company, Schenectady, NY 12345 (US)
(72) Inventor: Dragani , Patrick B., Whitefish Bay Wisconsin 53217 (US)
(74) Representative: Illingworth-Law, William Illingworth

(57) **Abstract**

A two sided cleansing wipe (10) has a first side (14) treated with a cleaner and a second side (16) treated with a disinfectant. The wipe is used to first apply the cleaner to the surface and thereafter apply a disinfectant. The first side may contain a color indicator to show the extent to which the surface has been cleaned. The indicator is removed during application of the disinfectant. The cleansing wipe finds use in cleaning and disinfecting medical equipment.

## Description

### FIELD OF THE INVENTION

The present invention relates to a wipe product having a cleaning agent on one side and a disinfectant on the other side. While not so limited, the invention finds particular utility in cleaning and disinfecting electronic medical equipment, including that used for collecting data from a patient.

### BACKGROUND OF THE INVENTION

In the medical field, many pieces of medical equipment directly interact with the patient. For example, medical electronics collect data from the patient usually through disposable electrodes placed on the patient's skin and this data is sent to the electronic device through flexible, reusable data cables. Because these data cables form the link between the patient and the electronic monitoring devices they are susceptible to contamination by many types of bacteria and spores present in the air or on the patient and/or contamination by organic matter such as mucous, food, or fecal matter from the patient. Therefore, these reusable cables must be cleaned and disinfected any time a clinician believes that a cable may have become contaminated, or otherwise at regular intervals.

Currently, a wide variety of cleansers and disinfectants are used by clinicians on user interfaces and data cables of electronic monitoring devices. These cleansers and disinfectants vary in chemical composition and reactivity, but many of them also include solvents that keep the active chemical ingredients in a liquid form. However, some of the cleaners, disinfectants, and solvents currently used are known to adversely react with the resins and plastics from which the devices to be cleaned are constructed, resulting in damage to the products. This is especially true for flexible data cables, as the use of improper cleaners and disinfectants on the cables may destroy their flexible quality making them rigid and therefore cracking easily. Additionally, the use of the improper disinfectants may cause the data transmission cables to short circuit providing improper reading and collection of patient biopotential quantities.

Proper cleaning of a medical device prior to being disinfected by a clinician is also important. If a clinician does not properly clean the medical device prior to disinfecting it, matter remaining on the medical device or data cables can break down the disinfectant when it is applied to the medical device or data cable. This may render the disinfectant ineffective and leave the medical device or data cable still contaminated.

A wipe product lends itself to removing contamination from medical devices, including data cables, and it is therefore desirable to have a wipe product for cleansing medical equipment that contains both a chemical cleaner and a chemical disinfectant. It is further desirable to provide a wipe product to clean and disinfect equipment that can provide a ready confirmation to a clinician that the product has been both cleaned and disinfected in an adequate manner. A proper wipe product will also help in the standardization of the techniques used by clinicians to clean and disinfect medical equipment, including data cables, as the proper chemical cleanser and chemical disinfectant can be included on the same wipe.

Briefly, an embodiment of the present invention is a two sided cleansing wipe that has one side treated with a chemical cleaner and a second side treated with a disinfectant chemical. The proper combinations of cleanser, disinfectant and any solvents for same can be provided in the wipes so that they will not harm the equipment upon which they are to be used.

In a further embodiment, the cleansing side of the wipe is additionally treated with a colored dye or indicator that is applied to the medical equipment and data cables during cleaning to indicate to the clinician the areas of the equipment that have been cleaned. This dye or indicator is removed by the disinfectant side of the wipe when the clinician disinfects the piece of equipment to ensure that those areas have been disinfected.

### BRIEF DESCRIPTION OF THE DRAWINGS

Embodiments of the present invention will now be described, by way of example only, with reference to the accompanying drawings, in which:
Fig. 1 depicts a flexible data cable with which the cleansing wipe of the present invention may be used.
Fig. 2 is a perspective view of a first embodiment of the present invention.
Fig. 3 is a cross-sectional view taken along line 3-3 of Fig. 2.
Fig. 4 shows a manner in which a wipe of the present invention may be folded for storage.
Fig. 5 shows a means for storing and dispensing the present invention.
Fig. 6 is another means for storing wipes of the present invention.
Fig. 7 depicts apparatus for determining that a piece of medical equipment has been cleaned and disinfected.
Fig. 8 depicts other apparatus for determining that a piece of medical equipment has been cleaned and disinfected.

### DETAILED DESCRIPTION OF THE INVENTION

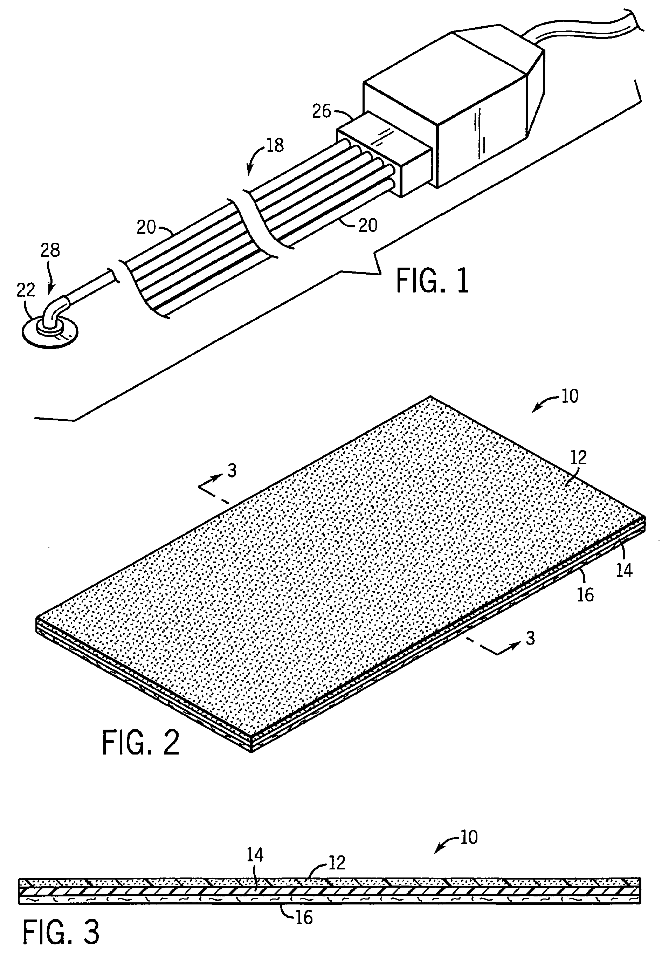

An embodiment of the present invention is a cleansing wipe for the cleaning and disinfection of medical equipment as well as a system of using this wipe to clean medical equipment. Embodiments of the present invention may be used to clean and disinfect medical equipment 18 including the flexible data cables 20 shown in Fig. 1.

Medical equipment 18 with a flexible data cable 20 connects to a plurality of electrodes, one of which is shown at 22, that are affixed to a patient. Flexible data cable 20 generally extends between a machine end 26 and a plurality of patient ends 28 connected to a plurality of electrodes 22. The flexible data cable 20 forms an integral link between the patient and the electronic medical equipment. By using inexpensive, disposable electrodes to obtain data from the patient, much of the potential contamination of the system is eliminated by disposing of these electrodes 22 after their use. However, because the flexible data cables 20 are in proximal relation to the patient the flexible data cables 20, especially the patient ends 28, are susceptible to being contaminated by the patient. Additionally, these cables may be contaminated from the bacteria and/or viruses present in the air and surrounding body portion of the patient. Therefore, it is necessary to clean and disinfect these reusable flexible data cables before they are used with a new patient so as not to spread the contamination among patients within a clinic or hospital.

Currently a wide variety of treatment products, treatment methods, and treatment frequency are used among clinicians to ensure that the medical devices themselves are not spreading the diseases that the clinicians are trying to fight. As noted above, this variety in treatments and methods has adverse effects upon the electronic devices themselves as well as on the quality of the disinfection that is performed.

It is preferable for proper disinfection that the surface to be disinfected is first properly cleaned to remove any organic or other matter that may be present on the flexible data cables or the electronic device because of potential contamination from these sources and because the presence of these contaminants may break down and render ineffective the disinfectant chemical when it is applied to the surface of the cables. To be effective, a chemical cleaner must be applied thoroughly and for a proper period of time. Without the proper application of the cleaner, the desired disinfectant results may not be achieved.

The improper combination of chemical cleaners and disinfectant chemicals may result in the damage or destruction of the flexible data cables, the underlying data wires, or the electronic device itself. This damage may be caused by a wide range of chemical interactions between the cleaning and disinfecting chemicals or solvents and the materials used to construct the flexible data cables. Specifically, many organic solvents, for example Butyl Cellosolve (a-butoxy ethanol), are known to be damaging to plastic and rubber products, especially polycarbonate plastic resins such as Lexan. These chemicals react with the plastic resins to break down the plasticizers in the plastics. This can cause them to become weak. Another effect of the loss of plasticizers is that the data cables lose their flexible quality thus rendering them rigid and susceptible to cracking. Also, the chemicals or solvents may interact with the metals used for structure (ferrous) or data transmission (copper) in the electronic devices. These interactions may cause the salt formed with the chlorides in the cleaners, disinfectants, or solvents to build up bridges of conductivity within the data transmission wires inside of the data cables resulting in incorrect data collection and patient monitoring results.

The cleansing wipe of the present invention is depicted in Fig. 2 and Fig. 3 to show its general shape and construction. Turning to Fig. 3 which is a cross-sectional view of the present invention taken along the line 3-3 of Fig. 2, the cleansing wipe 10 is of a layered construction comprising three layers, a chemical cleaner layer 12, a separator layer 14, and a disinfectant layer 16. The cleaner layer 12 and the disinfectant layer 16 are depicted as being constructed of foam and of non-woven material, respectively, but it is appreciated that these or other constructions may be used for either chemically treated side of wipe 10 or that an alternative material with absorbent properties such as a woven material may also be used in the construction of the present invention. Cleaner layer 12 contains a cleaning agent appropriate for equipment 18 and disinfectant layer 16 contains an appropriate disinfectant. The cleaning agent and the disinfectant may be retained in the layers by capillary action or other appropriate mechanism such as frangible encapsulation. One or both of layers 12 and 16 may contain colorization or other distinguishing characteristics to differentiate the two sides of the wipe.

The separator layer 14 is a thin flexible barrier layer disposed between the cleaner layer and the disinfectant layer. This may be formed of a plastic material, but it is appreciated that alternative substances that fulfill the function of limiting the interaction of the chemical cleaner and the disinfecting chemical on the respective sides of the wipe may be used in the construction of the present invention or that the barrier layer may be eliminated in the appropriate case.

The present invention as depicted in Fig. 3 addresses each of the problems noted above in a novel way. By the combination of a chemical cleaner on one side 12 and a disinfectant chemical on the other side 16 of the same two-sided cleansing wipe 10, the clinician will be encouraged to use the cleaning side of the two-sided cleansing wipe 10 first to properly clean the medical equipment to be disinfected and then to use the disinfectant chemical treated side 16 to properly disinfect the equipment.

The two-sided cleansing wipe 10 can provide a plurality of configurations with different combinations of chemical cleaner sides 12 and disinfectant chemical sides 16 so that the proper combination of chemical cleaners, disinfectant chemicals, as well as cleansing wipe material may be utilized for each piece of equipment 18. Both the cleaner and disinfectant should be non-injurious to the medical equipment on which the cleansing wipe is being used. Also, the cleaner and disinfectant should be chemically compatible so that no deleterious by-products are created when the cleaner and disinfectant come in contact. Suitable combinations of cleaners and disinfectants for use with medical equipment such as data cables include pairing many of the available antibacterial soaps with disinfectants such as Virex 256 available from S.C. Johnson or Cavicide available from Metrex.

The clinician is further encouraged to properly clean and disinfect the entire surface of medical equipment 18 in another embodiment of the invention. In this embodiment, the chemical cleaning side 12 of the cleansing wipe additionally contains a dye. A dye visible to a clinician under ordinary light may be used. Or, a dye that exhibits coloration when exposed to ultraviolet (UV) light may be used. As the clinician uses the cleaning side 12 of the two-sided cleansing wipe 10 to clean the medical device 18, the dye is transferred from the cleaning side 12 to the medical device that is being cleaned. The clinician would expose the medical device 18 that is being cleaned to ordinary or UV light as the clinician uses the chemical cleaner treated side 12 of the two-sided cleansing wipe or expose the medical device to light after the cleaning is deemed to be complete. This allows a visual determination of what areas of the medical device have been cleaned and the areas, if any, remaining to be cleaned.

When the entire medical device has been thoroughly cleaned by the chemical cleaner side 12 of the two-sided cleansing wipe 10, the dye will have been spread to all appropriate parts of the medical device, thus indicating the medical device has been thoroughly cleaned when examined under the appropriate light source.

Then the clinician turns the two-sided cleansing wipe 10 over and uses the disinfectant side 16 to disinfect medical device 18. As the disinfectant is applied on the medical device, the disinfectant side 16 will remove the dye. This will aid the clinician in determining what areas have been disinfected and what areas still need to be treated. When the dye has been completely removed, it indicates that disinfectant has been applied to all areas that were previously cleaned. This helps to ensure that the medical equipment has been both cleaned and disinfected.

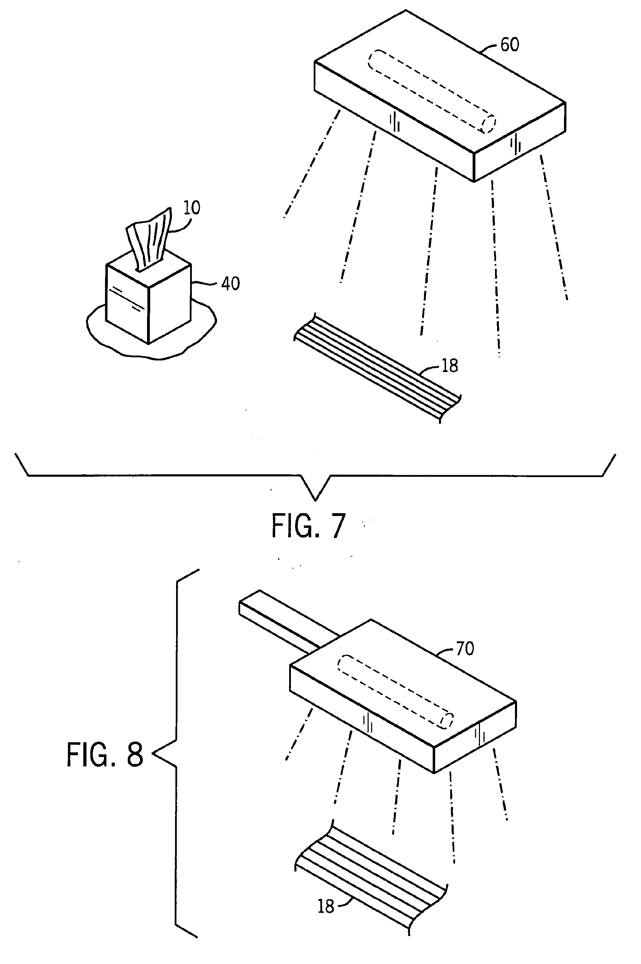

UV light, either a stationary UV light source 60 as depicted in Fig. 7 or a hand held UV light source 70 as depicted in Fig. 8 may be used in the steps of this process.

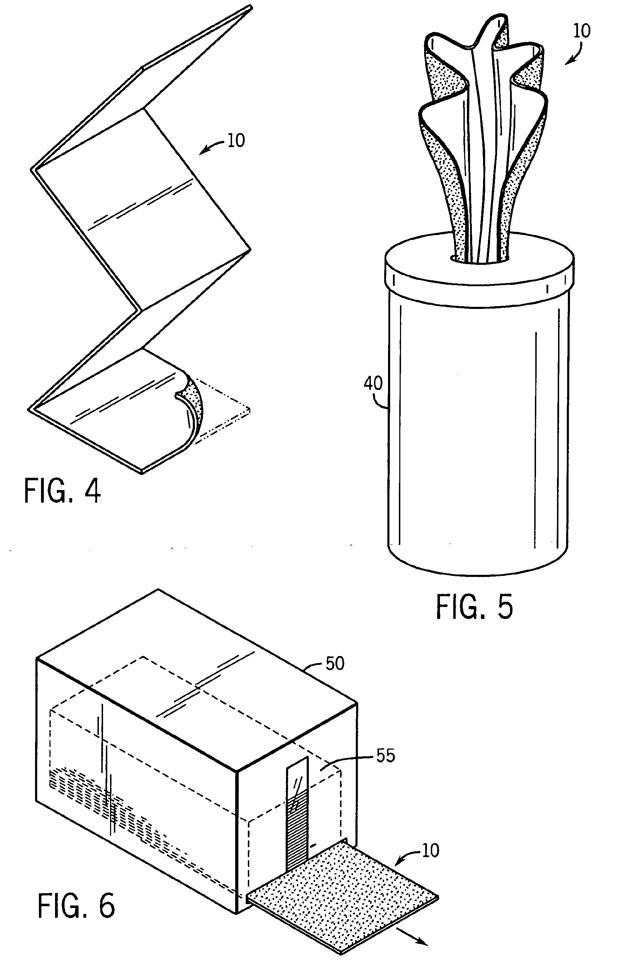

To further aid the clinician by encouraging the proper frequency of medical equipment cleaning and disinfecting, while using the correct combination of chemical cleaner and disinfectant for that particular electronic device, the two-sided cleansing wipes 10 may be packaged in a dispensing means in a way that they may be conveniently associated with a particular piece of equipment 18. Figs. 4-6 depict alternative embodiments of the packaging and storing the cleansing wipes.

Fig. 4 depicts a plurality of two-sided cleansing wipes in accordion style folded fashion so that the chemical treatments on the opposite sides of the two-sided cleansing wipe 10 would not come in contact with possible adverse consequences. The wipes so folded may form a stack that can be placed in a suitable dispensing means, if desired. Fig. 5 depicts a dispensing means similar to that of a typical baby-wipe canister 40 where individual sheets of the two-sided cleansing wipe 10 may be pulled from the top of the canister. Fig. 6 depicts a dispensing means for the two-sided cleansing wipe wherein the wipes 10 are stacked in a gravity based dispenser 50 and taken in turn from the bottom of the dispenser as needed by the clinician. The wipes may be individually packaged or accordion folded and perforated at the folds for separation. The dispensing means of Fig. 6 would also have an indicator means 55, shown as a clear window for viewing the remaining wipes, so the clinician may determine when the supply of two-sided cleansing wipes had been depleted and needed to be replenished.

Various alternatives and embodiments are contemplated as being within the scope of the following claims particularly pointing out and distinctly claiming the subject matter regarded as the invention.

### PARTS LIST

- 10.: Cleansing Wipe
- 12.: Chemical Cleaner Layer
- 14.: Separator Layer
- 16.: Disinfectant Layer
- 18.: Medical Equipment
- 20.: Flexible Data Cable
- 22.: Electrode
- 40.: Baby-Wipe Canister Dispensing Means
- 50.: Gravity Based Dispenser
- 55.: Indicator
- 60.: Stationary UV Light Source
- 70.: Hand-held UV Light Source

## Claims

1. A two-sided cleansing wipe (10) for medical equipment comprising:
a first side comprising a first layer (14) having a cleaner for application to the medical equipment to clean same, said cleaner being non-injurious to the medical equipment when applied to same; and
an opposite, second treated side comprising a second layer (16) having a disinfectant for application to the medical equipment to disinfect same, said disinfectant being non-injurious to the medical equipment when applied to same.

2. The cleansing wipe of claim 1 wherein said wipe has a third layer (14) intermediate said first and second layers for separating said first and second layers.

3. The cleansing wipe of claim 1 or 2 wherein at least one of said layers of said cleansing wipe is formed of foam, non-woven material, or woven material.

4. The cleansing wipe of claim 1 wherein at least one side of said cleansing wipe contains visual identification means.

5. The cleansing wipe according to claim 1 wherein said cleaner contains a dye preferably one that is apparent when exposed to ultraviolet light, for application to said medical equipment.

6. The cleansing wipe according to claims 1-5 wherein a plurality of wipes (10) are connected in a strip.

7. A method of cleaning and disinfecting medical equipment, comprising the steps of:
engaging a surface of the medical equipment with a first side of a cleansing wipe to apply a cleaner to the surface that is non-injurious to the medical equipment;
turning the wipe over; and
engaging the surface of the medical equipment with a second side of the cleansing wipe to apply a disinfectant to the surface that is non-injurious to the equipment..

8. A method in accordance with claim 7 further defined as using the first side of the wipe to spread a visual indicator on the surface of the medical equipment.

9. A method in accordance with claim 8 further defined as using the second side of the wipe to remove the visual indicator from the surface of the medical equipment.

10. A method in accordance with claim 8 wherein the visual indicator is a UV indicator and comprising the step of exposing the surface of the medical device to UV light following use of the first side of the cleansing wipe.
